# EUROPEAN PATENT SPECIFICATION

(11) **EP 1 723 143 B1**
(45) Date of publication and mention of the grant of the patent: **18.02.2009**
(21) Application number: 05714101.2
(22) Date of filing: 25.02.2005
(51) Int. Cl.: C07D 413/12

(54) **AMINO CYCLOPROPANE CARBOXAMIDE DERIVATIVES AS BRADYKININ ANTAGONISTS**
AMINOCYCLOPROPANCARBONSÄUREAMIDDERIVATE ALS BRADYKININANTAGONISTEN
DERIVES D AMINO CYCLOPROPANE CARBOXAMIDE UTILISES COMME ANTAGONISTES DE LA BRADYKININE

(30) Priority: 02.03.2004 US 549379 P
(43) Date of publication of application: 22.11.2006
(73) Proprietor: Merck & Co., Inc., Rahway, NJ 07065-0907 (US)
(72) Inventor: BOCK, Mark, G., Rahway, NJ 07065-0907 (US); FENG, Dong-Mei, Rahway, NJ 07065-0907 (US); KUDUK, Scott, Rahway, NJ 07065-0907 (US)
(74) Representative: Buchan, Gavin MacNicol
(86) International application number: PCT/US2005/006230
(87) International publication number: WO 2005/085198

(56) References cited:
- WO-A-03/065789
- WO-A-03/066577
- WO-A-20/04019868
- WO-A-20/05016886

## Description

### BACKGROUND OF THE INVENTION

Bradykinin ("BK") is a kinin which plays an important role in the pathophysiological processes accompanying acute and chronic pain and inflammation. Bradykinin (BK), like other kinins, is an autacoid peptide produced by the catalytic action of kallikrein enzymes on plasma and tissue precursors termed kininogens. The biological actions of BK are mediated by at least two major G-protein-coupled BK receptors termed B1 and B2. It is generally believed that B2 receptors, but not B1 receptors, are expressed in normal tissues and that inflammation, tissue damage or bacterial infection can rapidly induce B1 receptor expression. This makes the B1 receptor a particularly attractive drug target. The putative role of kinins, and specifically BK, in the management of pain and inflammation has provided the impetus for developing potent and selective BK antagonists. In recent years, this effort has been heightened with the expectation that useful therapeutic agents with analgesic and anti-inflammatory properties would provide relief from maladies mediated through a BK receptor pathway (see e.g., M.G. Bock and J. Longmore, Current Opinion in Chem. Biol., 4:401406(2000)). Accordingly, there is a need for novel compounds that are effective in blocking or reversing activation of bradykinin receptors. Such compounds would be useful in the management of pain and inflammation, as well as in the treatment or prevention of diseases and disorders mediated by bradykinin; further, such compounds are also useful as research tools (*in vivo* and *in vitro*).

WO 2005/016886 and WO 2005/019868 (which are prior art for the purposes of Art. 54(3) EPC), WO 03/ 065789 and WO 03/066577 disclose certain aminocycloalkane carboxamide derivatives as bradykinin antagonists.

### SUMMARY OF THE INVENTION

The present invention provides aminocyclopropanecarboxamide derivatives which are bradykinin antagonists or inverse agonists, pharmaceutical compositions containing such compounds, and methods of using them as therapeutic agents.

### DETAILED DESCRIPTION OF THE INVENTION

The present invention provides for a compound of formula Ia and pharmaceutically acceptable salts thereof: 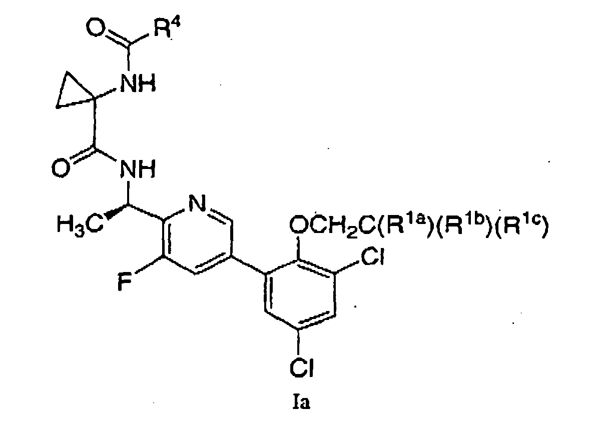 wherein R^{1a}, R^{1b} and R^{1c} are each independently selected from hydrogen and fluorine; R4 is (a) optionally substituted 5-membered heteroaromatic ring having a ring heteroatom selected from N, O and S, and optionally having up to 3 additional ring nitrogen atoms; or (b) optionally substituted 6-membered heteroaromatic ring containing from 1 to 3 ring nitrogen atoms and N-oxides thereof; wherein the substitutent is 1 to 2 groups independently selected from halogen, C₁₋₄alkyl optionally substituted with C₁₋₄alkoxy, C₁₋₄alkoxy, hydroxy, C₃₋₆ cycloalkyl, and CF₃.

In one embodiment are compounds wherein R⁴ is selected from optionally substituted isoxazolyl, optionally substituted oxazolyl, optionally substituted isothiazolyl, optionally substituted thiazolyl, optionally substituted pyridazinyl and optionally substituted pyrazinyl, wherein the substituent is 1 to 2 groups selected from halogen, C₁₋₄alkyl optionally substituted with C₁₋₄alkoxy, C₁₋₄alkoxy, hydroxy, and CF₃.

In a second embodiment are compounds wherein R⁴ is selected from 3-chloro-5-isoxazolyl, 3-methoxy-5-isoxazolyl, 3-ethoxy-5-isoxazolyl, arid 3-methyl-5-isoxazolyl.

In a third embodiment are compounds wherein R⁴ is selected from 5-thiazolyl optionally substituted at the 2-position with chlorine, bromine, or methoxymethyl.

A second aspect of the present invention provides a pharmaceutical composition comprising a therapeutically effective amount of a compound of formula Ia or a pharmaceutically acceptable salt thereof, and a pharmaceutically acceptable carrier.

A third aspect of the present invention provides the use of a compound of formula Ia or a pharmaceutically acceptable salt thereof for the manufacture of a medicament for the treatment of conditions mediated by bradykinin B I receptor. In one subset said condition is pain including inflammatory and neuropathic pain.

Unless otherwise stated, the following terms have the meanings indicated below:
"Alkyl" as well as other groups having the prefix "alk" such as, for example, alkoxy, alkanoyl, and the like, means carbon chains which may be linear or branched or combinations thereof. Examples of alkyl groups include methyl, ethyl, propyl, isopropyl, butyl, sec- and tert-butyl, pentyl, hexyl and the like.
"Cycloalkyl" means carbocycles containing no heteroatoms, and includes mono-, bi- and tricyclic saturated carbocycles, as well as fused ring systems. Such fused ring systems can include one ring that is partially or fully unsaturated such as a benzene ring to form fused ring systems such as benzofused carbocycles. Cycloalkyl includes such fused ring systems as spirofused ring systems. Examples of cycloalkyl include cyclopropyl, cyclobutyl, cyclopentyl, cyclohexyl, decahydronaphthalene, adamantane, indanyl, indenyl, fluorenyl, 1,2,3,4-tetrahydronaphthalene and the like.
"Haloalkyl" means an alkyl radical as defined above wherein at least one and up to all of the hydrogen atoms are replaced with a halogen. Examples of such haloalkyl radicals include chloromethyl, 1-bromoethyl, fluoromethyl, difluoromethyl, trifluoromethyl, 2,2,2-trifluoroethyl and the like.
"Halogen" means fluorine, chlorine, bromine and iodine.
"5-Membered heteroaromatic ring" includes, without limitation, pyrrole, imidazole, pyrazole, 1,2,3-triazole, 1,2,4-triazole, tetrazole, furan, thiophene, thiazole, oxazole, isothiazole, isoxazole, 1,2,4-oxadiazole, 1,2,5-oxadiazole, 1,2,4-thiadiazole, and 1,2,5-thiadiazole.
"6-Membered heteroaromatic ring" includes, without limitation, pyridine, pyrimidine, pyridazine, pyrazine, and triazine.

### Optical Isomers - Diastercomers - Geometric Isomer - Tautomers.

Compounds described herein contain an asymmetric center and may thus exist as enantiomers. Where the compounds according to the invention possess two or more asymmetric centers, they may additionally exist as diastereomers. The present invention includes all such possible stereoisomers as substantially pure resolved enantiomers, racemic mixtures thereof, as well as mixtures of diastereomers. The above Formula I is shown without a definitive stereochemistry at certain positions. The present invention includes all stereoisomers of Formula Ia and pharmaceutically acceptable salts thereof. Diastereoisomeric pairs of enantiomers may be separated by, for example, fractional crystallization from a suitable solvent, and the pair of enantiomers thus obtained may be separated into individual stereoisomers by conventional means, for example by the use of an optically active acid or base as a resolving agent or on a chiral HPLC column. Further, any enantiomer or diastereomer of a compound of the general Formula I may be obtained by stereospecific synthesis using optically pure starting materials or reagents of known configuration.

Some of the compounds described herein contain olefinic double bonds, and unless specified otherwise, are meant to include both E and Z geometric isomers.

Some of the compounds described herein may exist with different points of attachment of hydrogen, referred to as tautomers. Such an example may be a ketone and its enol form known as keto-enol tautomers. The individual tautomers as well as mixture thereof are encompassed with compounds of Formula I.

### Salts.

The term "pharmaceutically acceptable salts" refers to salts prepared from pharmaceutically acceptable non-toxic bases or acids. When the compound of the present invention is acidic, its corresponding salt can be conveniently prepared from pharmaceutically acceptable non-toxic bases, including inorganic bases and organic bases. Salts derived from such inorganic bases include aluminum, ammonium, calcium, copper (ic and ous), ferric, ferrous, lithium, magnesium, manganese (ic and ous), potassium, sodium, zinc and the like salts. Preferred are the ammonium, calcium, magnesium, potassium and sodium salts. Salts prepared from pharmaceutically acceptable organic non-toxic bases include salts of primary, secondary, and tertiary amines derived from both naturally occurring and synthetic sources. Pharmaceutically acceptable organic non-toxic bases from which salts can be formed include, for example, arginine, betaine, caffeine, choline, N,N^{'}-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, dicyclohexylamine, lysine, methylglucamine, morpholine, piperazine, piperidine, polyamine resins, procaine, purines, theobromine, triethylamine, trimethylamine, tripropylamine, tromethamine and the like.

When the compound of the present invention is basic, its corresponding salt can be conveniently prepared from pharmaceutically acceptable non-toxic inorganic and organic acids. Such acids include, for example, acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid and the like. Preferred are citric, hydrobromic, hydrochloric, maleic, phosphoric, sulfuric, and tartaric acids.

### Pharmaceutical Compositions.

Another aspect of the present invention provides pharmaceutical compositions which comprises a compound of Formula Ia, or a pharmaceutically acceptable salt thereof, and a pharmaceutically acceptable carrier. The term "composition", as in pharmaceutical composition, is intended to encompass a product comprising the active ingredient(s), and the inert ingredient(s) (pharmaceutically acceptable excipients) that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a compound of Formula Ia, additional active ingredient(s), and pharmaceutically acceptable excipients.

The pharmaceutical compositions of the present invention comprise a compound represented by Formula Ia (or pharmaceutically acceptable salts thereof) as an active ingredient, a pharmaceutically acceptable carrier and optionally other therapeutic ingredients or adjuvants. The compositions include compositions suitable for oral, rectal, topical, and parenteral (including subcutaneous, intramuscular, and intravenous) administration, although the most suitable route in any given case will depend on the particular host, and nature and severity of the conditions for which the active ingredient is being administered. The pharmaceutical compositions may be conveniently presented in unit dosage from and prepared by any of the methods well known in the art of pharmacy.

In practice, the compounds represented by Formula Ia, or pharmaceutically acceptable salts thereof, of this invention can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). Thus, the pharmaceutical compositions of the present invention can be presented as discrete units suitable for oral administration such as capsules, cachets or tablets each containing a predetermined amount of the active ingredient. Further, the compositions can be presented as a powder, as granules, as a solution, as a suspension in an aqueous liquid, as a nonaqueous liquid, as an oil-in-water emulsion or as a water-in-oil liquid emulsion. In addition to the common dosage forms set out above, the compound represented by Formula Ia, or pharmaceutically acceptable salts thereof, may also be administered by controlled release means and/or delivery devices. The compositions may be prepared by any of the methods of pharmacy. In general, such methods include a step of bringing into association the active ingredient with the carrier that constitutes one or more necessary ingredients. In general, the compositions are prepared by uniformly and intimately admixing the active ingredient with liquid carriers or finely divided solid carriers or both. The product can then be conveniently shaped into the desired presentation.

Thus, the pharmaceutical compositions of this invention may include a pharmaceutically acceptable carrier and a compound or a pharmaceutically acceptable salt of Formula Ia. The compounds of Formula I, or pharmaceutically acceptable salts thereof, can also be included in pharmaceutical compositions in combination with one or more other therapeutically active compounds.

The pharmaceutical carrier employed can be, for example, a solid, liquid, or gas. Examples of solid carriers include lactose, terra alba, sucrose, tale, gelatin, agar, pectin, acacia, magnesium stearate, and stearic acid. Examples of liquid carriers are sugar syrup, peanut oil, olive oil, and water. Examples of gaseous carriers include carbon dioxide and nitrogen.

In preparing the compositions for oral dosage form, any convenient pharmaceutical media may be employed. For example, water, glycols, oils, alcohols, flavoring agents, preservatives, coloring agents and the like may be used to form oral liquid preparations such as suspensions, elixirs and solutions; while carriers such as starches, sugars, microcrystalline cellulose, diluents, granulating agents, lubricants, binders, disintegrating agents, and the like may be used to form oral solid preparations such as powders, capsules and tablets. Because of their ease of administration, tablets and capsules are the preferred oral dosage units whereby solid pharmaceutical carriers are employed. Optionally, tablets may be coated by standard aqueous or nonaqueous techniques

A tablet containing the composition of this invention may be prepared by compression or molding, optionally with one or more accessory ingredients or adjuvants. Compressed tablets may be prepared by compressing, in a suitable machine, the active ingredient in a free-flowing form such as powder or granules, optionally mixed with a binder, lubricant, inert diluent, surface active or dispersing agent. Molded tablets may be made by molding in a suitable machine, a mixture of the powdered compound moistened with an inert liquid diluent. Each tablet preferably contains from about 0.1mg to about 500mg of the active ingredient and each cachet or capsule preferably containing from about 0.1 mg to about 500mg of the active ingredient.

Pharmaceutical compositions of the present invention suitable for parenteral administration may be prepared as solutions or suspensions of the active compounds in water. A suitable surfactant can be included such as, for example, hydroxypropylcellulose. Dispersions can also be prepared in glycerol, liquid polyethylene glycols, and mixtures thereof in oils. Further, a preservative can be included to prevent the detrimental growth of microorganisms.

Pharmaceutical compositions of the present invention suitable for injectable use include sterile aqueous solutions or dispersions. Furthermore, the compositions can be in the form of sterile powders for the extemporaneous preparation of such sterile injectable solutions or dispersions. In all cases, the final injectable form must be sterile and must be effectively fluid for easy syringability. The pharmaceutical compositions must be stable under the conditions of manufacture and storage; thus, preferably should be preserved against the contaminating action of microorganisms such as bacteria and fungi. The carrier can be a solvent or dispersion medium containing, for example, water, ethanol, polyol (e.g. glycerol, propylene glycol and liquid polyethylene glycol), vegetable oils, and suitable mixtures thereof.

Pharmaceutical compositions of the present invention can be in a form suitable for topical use such as, for example, an aerosol, cream, ointment, lotion, dusting powder, or the like. Further, the compositions can be in a form suitable for use in transdermal devices. These formulations may be prepared, utilizing a compound represented by Formula Ia of this invention, or pharmaceutically acceptable salts thereof, via conventional processing methods. As an example, a cream or ointment is prepared by mixing hydrophilic material and water, together with about 5 wt% to about 10 wt% of the compound, to produce a cream or ointment having a desired consistency.

Pharmaceutical compositions of this invention can be in a form suitable for rectal administration wherein the carrier is a solid. It is preferable that the mixture forms unit dose suppositories. Suitable carriers include cocoa butter and other materials commonly used in the art. The suppositories may be conveniently formed by first admixing the composition with the softened or melted carrier(s) followed by chilling and shaping in moulds.

In addition to the aforementioned carrier ingredients, the pharmaceutical formulations described above may include, as appropriate, one or more additional carrier ingredients such as diluents, buffers, flavoring agents, binders, surface-active agents, thickeners, lubricants, preservatives (including anti-oxidants) and the like. Furthermore, other adjuvants can be included to render the formulation isotonic with the blood of the intended recipient. Compositions containing a compound described by Formula I, or pharmaceutically acceptable salts thereof, may also be prepared in powder or liquid concentrate form.

The following are examples of representative pharmaceutical dosage forms for the compounds of Formula Ia:

| Inj. Suspension (I.M.) | mg/mL | Tablet | mg/tab. | Capsule | mg/cap. |
|---|---|---|---|---|---|
| Cmpd of Formula Ia | 10 | Cmpd of Formula Ia | 25 | Cmpd of Formula Ia | 25 |
| Methylcellulose5.0 | | Microcryst. Cellulose | 415 | Lactose Powder573.5 | |
| Tween 80 | 0.5 | Povidone | 14.0 | Magnesium Stearate | 1.5 |
| Benzyl alcohol 9.0 | | Pregelatinized Starch | 43.5 | | 600 |
| Benzalkonium chloride | 1.0 | Magnesium Stearate | 2.5 | | |
| Water for injection to a total volume of 1 mL | | | 500 | | |

### Utilities

Compounds of this invention are antagonists or inverse agonists of bradykinin receptor, in particular the bradykinin B1 receptor, and as such are useful in the treatment and prevention of diseases and conditions mediated through the bradykinin receptor pathway such as pain and inflammation. The compounds would be effective in the treatment or prevention of pain including, for example, visceral pain (such as pancreatitis, interstitial cystitis, renal colic, prostatitis, chronic pelvic pain), neuropathic pain (such as postherpetic neuralgia, acute zoster pain, nerve injury, the "dynias", e.g., vulvodynia, phantom limb pain, root avulsions, radiculopathy, painful traumatic mononeuropathy, painful entrapment neuropathy, carpal tunnel syndrome, ulnar neuropathy, tarsal tunnel syndrome, painful diabetic neuropathy, painful polyneuropathy, trigeminal neuralgia), central pain syndromes (potentially caused by virtually any lesion at any level of the nervous system including but not limited to stroke, multiple sclerosis, spinal cord injury), and postsurgical pain syndromes (eg, postmastectomy syndrome, postthoracotomy syndrome, stump pain)), bone and joint pain (osteoarthritis), spine pain (e.g., acute and chronic low back pain, neck pain, spinal stenosis), shoulder pain, repetitive motion pain, dental pain, sore throat, cancer pain, myofascial pain (muscular injury, fibromyalgia), postoperative, periopcrative pain and preemptive analgesia (including but not limited to general surgery, orthopedic, and gynecological), chronic pain, dysmenorrhea (primary and secodnary), as well as pain associated with angina, and inflammatory pain of varied origins (e.g. osteoarthritis, rheumatoid arthritis, rheumatic disease, teno-synovitis and gout, ankylosing spondylitis, bursitis).

Further, the compounds of this invention can also be used to treat hyperreactive airways and to treat inflammatory events associated with airways disease e.g. asthma including allergic asthma (atopic or non-atopic) as well as exercise-induced bronchoconstriction, occupational asthma, viral- or bacterial exacerbation of asthma, other non-allergic asthmas and "wheezy-infant syndrome". Compounds of the present invention may also be used to treat chronic obstructive pulmonary disease including emphysema, adult respiratory distress syndrome, bronchitis, pneumonia, allergic rhinitis (seasonal and perennial), and vasomotor rhinitis. They may also be effective against pneumoconiosis, including aluminosis, anthracosis, asbestosis, chalicosis, ptilosis, siderosis, silicosis, tabacosis and byssinosis.

Compounds of the present invention may also be used for the treatment of inflammatory bowel disease including Crohn's disease and ulcerative colitis, irritable bowel syndrome, pancreatitis, nephritis, cystitis (interstitial cystitis), uveitis, inflammatory skin disorders such as psoriasis and eczema, rheumatoid arthritis and edema resulting from trauma associated with burns, sprains or fracture, cerebral edema and angioedema. They may be used to treat diabetic vasculopathy, diabetic neuropathy, diabetic retinopathy, post capillary resistance or diabetic symptoms associated with insulitis (e.g. hyperglycemia, diuresis, proteinuria and increased nitrite and kallikrein urinary excretion). They may be used as smooth muscle relaxants for the treatment of spasm of the gastrointestinal tract or uterus. Additionally, they may be effective against liver disease, multiple sclerosis, cardiovascular disease, e.g. atherosclerosis, congestive heart failure, myocardial infarct; neurodegenerative diseases, eg. Parkinson's and Alzheimers disease, epilepsy, septic shock e.g. as anti-hypovolemic and/or anti-hypotensive agents, headache including cluster headache, migraine including prophylactic and acute use, stroke, closed head trauma, cancer, sepsis, gingivitis, osteoporosis, benign prostatic hyperplasia and hyperactive bladder. Animal models of these diseases and conditions are generally well known in the art, and may be suitable for evaluating compounds of the present invention for their potential utilities. Finally, compounds of the present invention are also useful as research tools (*in vivo* and *in vitro*).

The compounds of this invention are useful in the treatment of pain and inflammation by the administration of a tablet, cachet, or capsule each containing, for example, 0.1mg, 0.5mg, 1mg, 3mg, 5mg, 10mg, 25mg, 50mg, 100mg, 125mg, 250mg, or 500mg of a compound of this invention once every three to four hours, once, twice or three times a day, or (in an extended release formulation) once, twice or three times a week.

The compounds would be effective in the treatment or prevention of pain including, for example, bone and joint pain (osteoarthritis), repetitive motion pain, dental pain, cancer pain, myofascial pain (muscular injury, fibromyalgia), perioperative pain (general surgery, oral surgery, gynecological), neuropathic pain (post-herpetic neuralgia), and chronic pain by the administration of a tablet, cachet, or capsule each containing, for example, 0.1mg, 0.5mg, 1mg, 3mg, 5mg, 10mg, 25mg, 50mg, 100mg, 125mg, 250mg, or 500mg of a compound of this invention once every three to four hours, once, twice or three times a day, or (in an extended release formulation) once, twice or three times a week.

In particular, inflammatory pain such as, for example, inflammatory airways disease (chronic obstructive pulmonary disease) would be effectively treated by the compounds of this invention by the administration of a tablet, cachet, or capsule each containing, for example, 0.1mg, 0.5mg, 1mg, 3mg, 5mg, 10mg, 25mg, 50mg, 100mg, 125mg, 250mg, or 500mg of a compound of this invention once every three to four hours, once, twice or three times a day, or (in an extended release formulation) once, twice or three times a week.

Further, the compounds of this invention can additionally be used to treat asthma, inflammatory bowel disease, rhinitis, pancreatitis, cystitis (interstitial cystitis), uveitis, inflammatory skin disorders, rheumatoid arthritis and edema resulting from trauma associated with bums, sprains or fracture by the administration of a tablet, cachet, or capsule each containing, for example, 0.1mg, 0.5mg, 1mg, 3mg, 5mg, 10mg, 25mg, 50mg, 100mg, 125mg, 250mg, or 500mg of a compound of this invention once every three to four hours, once, twice or three times a day, or (in an extended release formulation) once, twice or three times a week.

They may be used subsequent to surgical intervention (e.g. as post-operative analgesics) and to treat inflammatory pain of varied origins (e.g. osteoarthritis, rheumatoid arthritis, rheumatic disease, teno-synovitis and gout) as well as for the treatment of pain associated with angina, menstruation or cancer by the administration of a tablet, cachet, or capsule each containing, for example, 0.1mg, 0.5mg, 1mg, 3mg, 5mg, 10mg, 25mg, 50mg, 100mg, 125mg, 250mg, or 500mg of a compound of this invention once every three to four hours, once, twice or three times a day, or (in an extended release formulation) once, twice or three times a week.

They may be used to treat diabetic vasculopathy, post capillary resistance or diabetic symptoms associated with insulitis (e.g. hyperglycemia, diuresis, proteinuria and increased nitrite and kallikrein urinary excretion) by the administration of a tablet, cachet, or capsule each containing, for example, 0.1mg, 0.5mg, 1mg, 3mg, 5mg, 10mg, 25mg, 50mg, 100mg, 125mg, 250mg, or 500mg of a compound of this invention once every three to four hours, once, twice or three times a day, or (in an extended release formulation) once, twice or three times a week.

They may be used to treat inflammatory skin disorders such as psoriasis and eczema by the administration of a tablet, cachet, or capsule each containing, for example, 0.1mg, 0.5mg, 1mg, 3mg, 5mg, 10mg, 25mg, 50mg, 100mg, 125mg, 250mg, or 500mg of a compound of this invention once every three to four hours, once, twice or three times a day, or (in an extended release formulation) once, twice or three times a week.

They may be used as smooth muscle relaxants for the treatment of spasm of the gastrointestinal tract or uterus or in the therapy of Crohn's disease, ulcerative colitis or pancreatitis by the administration of a tablet, cachet, or capsule each containing, for example, 0.1mg, 0.5mg, 1mg, 3mg, 5mg, 10mg, 25mg, 50mg, 100mg, 125mg, 250mg, or 500mg of a compound of this invention once every three to four hours, once, twice or three times a day, or (in an extended release formulation) once, twice or three times a week.

Such compounds may be used therapeutically to treat hyperreactive airways and to treat inflammatory events associated with airways disease e.g. asthma, and to control, restrict or reverse airways hyperreactivity in asthma by the administration of a tablet, cachet, or capsule each containing, for example, 0.1mg, 0.5mg, 1mg, 3mg, 5mg, 10mg, 25mg, 50mg, 100mg, 125mg, 250mg, or 500mg of a compound of this invention once every three to four hours, once, twice or three times a day, or (in an extended release formulation) once, twice or three times a week.

They may be used to treat intrinsic and extrinsic asthma including allergic asthma (atopic or non-atopic) as well as exercise-induced bronchoconstriction, occupational asthma, viral or bacterial exacerbated asthma, other non-allergic asthmas and "wheezy-infant syndrome" by the administration of a tablet, cachet, or capsule each containing, for example, 0.1mg, 0.5mg, 1mg, 3mg, 5mg, 10mg, 25mg, 50mg, 100mg, 125mg, 250mg, or 500mg of a compound of this invention once every three to four hours, once, twice or three times a day, or (in an extended release formulation) once, twice or three times a week.

They may also be effective against pneumoconiosis, including aluminosis, anthracosis, asbestosis, chalicosis, ptilosis, siderosis, silicosis, tabacosis and byssinosis was well as adult respiratory distress syndrome, chronic obstructive pulmonary or airways disease, bronchitis, allergic rhinitis, and vasomotor rhinitis by the administration of a tablet, cachet, or capsule each containing, for example, 0.1mg, 0.5mg, 1mg, 3mg, 5mg, 10mg, 25mg, 50mg, 100mg, 125mg, 250mg, or 500mg of a compound of this invention once every three to four hours, once, twice or three times a day, or (in an extended release formulation) once, twice or three times a week.

Additionally, they may be effective against liver disease, multiple sclerosis, atherosclerosis, Alzheimer's disease, septic shock e.g. as anti-hypovolemic and/or anti-hypotensive agents, cerebral edema, headache including cluster headache, migraine including prophylactic and acute use, closed head trauma, irritable bowel syndrome and nephritis by the administration of a tablet, cachet, or capsule each containing, for example, 0.1mg, 0.5mg, 1mg, 3mg, 5mg, 10mg, 25mg, 50mg, 100mg, 125mg, 250mg, or 500mg of a compound of this invention once every three to four hours, once, twice or three times a day, or (in an extended release formulation) once, twice or three times a week.

### Combination Therapy

Compounds of Formula Ia may be used in combination with other drugs that are used in the treatment/prevention/suppression or amelioration of the diseases or conditions for which compounds of Formula Ia are useful. Such other drugs may be administered, by a route and in an amount commonly used therefor, contemporaneously or sequentially with a compound of Formula Ia. When a compound of Formula I is used contemporaneously with one or more other drugs, a pharmaceutical composition containing such other drugs in addition to the compound of Formula Ia is preferred. Accordingly, the pharmaceutical compositions of the present invention include those that also contain one or more other active ingredients, in addition to a compound of Formula Ia. Examples of other active ingredients that may be combined with a compound of Formula Ia, either administered separately or in the same pharmaceutical compositions, include, but are not limited to: (1) morphine and other opiate receptor agonists including propoxyphene (Darvon) and tramadol; (2) non-steroidal antiinflammatory drugs (NSAIDs) including COX-2 inhibitors such as propionic acid derivatives (alminoprofen, benoxaprofen, bucloxic acid, carprofen, fenbufen, fenoprofen, fluprofen, flurbiprofen, ibuprofen, indoprofen, ketoprofen, miroprofen, naproxen, oxaprozin, pirprofen, pranoprofen, suprofen, tiaprofenic acid, and tioxaprofen), acetic acid derivatives (indomethacin, acemetacin, alclofenac, clidanac, diclofenac, fenclofenac, fenclozic acid, fentiazac, furofenac, ibufenac, isoxepac, oxpinac, sulindac, tiopinac, tolmetin, zidometacin, and zomepirae), fenamic acid derivatives (flufenamic acid, meclofenamic acid, mefenamic acid, niflumic acid and tolfenamic acid), biphenylcarboxylic acid derivatives (diflunisal and flufenisal), oxicams (isoxicam, piroxicam, sudoxicam and tenoxican), salicylates (acetyl salicylic acid, sulfasalazine) and the pyrazolones (apazone, bezpiperylon, feprazone, mofebutazone, oxyphenbutazone, phenylbutazone), and the coxibs (celecoxib, valecoxib, rofecoxib and etoricoxib); (3) corticosteroids such as betamethasone, budesonide, cortisone, dexamethasone, hydrocortisone, methylprednisolone, prednisolone, prednisone and triamcinolone; (4) histamine H1 receptor antagonists such as bromopheniramine, chlorpheniramine, dexchlorpheniramine, triprolidine, clemastine, diphenhydramine, diphenylpyraline, tripelennamine, hydroxyzine, methdilazine, promethazine, trimeprazine, azatadine, cyproheptadine, antazoline, pheniramine pyrilamine, astemizole, terfenadine, loratadine, cetirizine, desloratadine, fexofenadine and levocetirizine; (5) histamine H2 preceptor antagonists such as cimetidine, famotidine and ranitidine; (6) proton pump inhibitors such as omeprazole, pantoprazole and esomeprazole; (7) leukotriene antagonists and 5-lipoxygenase inhibitors such as zafirlukast, montelukast, pranlukast and zileuton; (8) drugs used for angina, myocardial ischemia including nitrates such as nitroglycerin and isosorbide nitrates, beta blockers such as atenolol, metoprolol, propranolol, acebutolol ,betaxolol, bisoprolol, carteolol, labetalol, nadolol, oxprenolol, penbutolol, pindolol, sotalol and timolol, and calcium channel blockers such as diltiazam, verapamil, nifedipine, bepridil, felodipine, flunarizinc, isradipine, nicardipine and nimodipine; (9) incontinence medications such as antimuscarinics, *e*.*g*., tolterodine and oxybutinin); (10) gastrointestinal antispasmodics (such as atropine, scopolamine, dicyclomine, antimuscarinics, as well as diphenoxylate); skeletal muscle relaxants (cyclobenzaprine, carisoprodol, chlorphenesin, chlorzoxazone, metaxalone, methocarbamol, baclofen, dantrolene, diazepam, or orphenadrine); (11) gout medications such as allopurinol, probenicid and colchicine; (12) drugs for rheumatoid arthritis such as methotrexate, auranofin, aurothioglucose and gold sodium thiomalate; (13) drugs for osteoporosis such as alendronate and raloxifene; decongestants such as pseudoephedrine and phenylpropanolamine; (14) local anesthetics; (15) anti-herpes drugs such as acyclovir, valacyclovir and famcyclovir; (16) anti-emetics such as ondansetron and granisetron; (17) migraine drugs such as the triptans (e.g. rizatriptan, sumatriptan), ergotamine, dihydroergotamine, CGRP antagonists, antidepressants (e.g., tricyclic antidepressants, serotonin-selective reuptake inhibitors, beta-adrenergic blockers); (18) VR1 antagonsits; (19) anticonvulsants (e.g., gabapentin, pregabalin, lamotrigine, topiramate, carbamazepine, oxcarbazepine, phenytoin); (20) glutamate antagonists (e.g., ketamine and other NMDA antagonists, NR2B antagonists); (21) acetaminophen; (22) CCR2 antagonists; (23) PDE4 antagonists.

### Biological Evaluation

### (a) Assessing the Affinity of Selected Compounds to Bind to the Bradykinin B1 or B2 Receptor

Radioligand binding assays are performed using membranes from CHO cells that stably express the human, rabbit, rat, or dog B 1 receptors or CHO cells that express the human B2 receptor. For all receptor types, cells are harvested from culture flasks in PBS/1mM EDTA and centrifuged at 1000xg for 10 minutes. The cell pellets are homogenized with a polytron in ice cold 20mM HEPES, 1mM EDTA, pH 7.4 (lysis buffer) and centrifuged at 20,000xg for 20 minutes. The membrane pellets are rehomogenized in lysis buffer, centrifuged again at 20,000xg and the final pellets are resuspended at 5mg protein/ml in assay buffer (120mM NaCl, 5mM KCI, 20mM HEPES, pH 7.4) supplemented with 1% BSA and frozen at -80°C.

On the day of assay, membranes are centrifuged at 14,000xg for 5 minutes and resuspended to the desired protein concentration in assay buffer containing 100nM enaliprilat, 140µg/mL bacitracin and 0.1% BSA. 3H-des-arg10, leu9 kallidin is the radioligand used for the human and rabbit B 1 receptors, 3H-des-arg10 kallidin is used for the rat and dog B1 receptors, and 3H-bradykinin is used to label the human B2 receptor.

For all assays, compounds are diluted from DMSO stock solutions with 4µL added to assay tubes for a final DMSO concentration of 2%. This is followed by the addition of 100µL radioligand and 100µL of the membrane suspension. Nonspecific binding for the B I receptor binding assays is determined using 1µM des-arg10 kallidin and nonspecific binding for the B2 receptor is determined with 1µM bradykinin. Tubes are incubated at room temperature (22°C) for 60 minutes followed by filtration using a Tomtec 96-well harvesting system. Radioactivity retained by the filter is counted using a Wallac Beta-plate scintillation counter.

The compounds of this invention have affinity for the B1 receptor in the above assay as demonstrated by results of less than 5µM. It is advantageous that the assay results be less than 1µM, even more advantageous for the results be less than 0.5µM. It is further advantageous that compounds of this invention have affinity for the bradykinin B1 receptor over the bradykinin B2 receptor; more advantageously, the affinity for the B1 receptor is at least 10 fold, and preferably over 100 fold, over that for the B2 receptor.

### (b) Assay for Bradykinin B1 Antagonists

B I agonist-induced calcium mobilization was monitored using a Fluorescence Imaging Plate Reader (FLIPR). CHO cells expressing the B1 receptor were plated in 96 or 384 well plates and allowed to incubate in Iscove's modified DMEM overnight. Wells were washed two times with a physiological buffered salt solution and then incubated with 4uM Fluo-3 for one hour at 37°C. The plates were then washed two times with buffered salt solution and 100uL of buffer was added to each well. Plates were placed in the FLIPR unit and allowed to equilibrate for two minutes. The test compound was then added in 50ul volumes followed five minutes later by 50ul of agonist (des-arg10 kallidin). Relative fluorescence peak heights in the absence and presence of antagonist were used to calculate the degree of inhibition of the B1 receptor agonist response by the test compound. Eight to ten concentrations of test compound were typically evaluated to construct an inhibition curve and determine IC50 values using a four-parameter nonlinear regression curve fitting routine.

### (c) Assay for Bradykinin Inverse Agonists

Inverse agonist activity at the human B1 receptor was evaluated using transiently transfected HEK293 cells. One day following transfection cell flasks were labeled overnight with 6uCi/ml [³H]myo-inositol. On the day of assay, the media was removed and the attached cells were gently rinsed with 2x20ml of phosphate-buffered saline. Assay buffer (HEPES buffered physiological salts, pH 7.4) was added and the cells were detached by tapping of the flask. The cells were centrifuged at 800xg for five minutes and resuspended at 1x10⁶ cells/ml in assay buffer supplemented with 10mM lithium chloride. After 10 minutes at room temperature, one-half ml aliquots were distributed to tubes containing test compound or vehicle. After an additional 10 minutes the tubes were transferred to a 37°C water bath for 30 minutes. The incubation was terminated by the addition of a 12% perchloric acid solution and the tubes were placed on ice for 30 minutes. The acid was then neutralized with KOH and the tubes centrifuged to pellet precipitated material. [³H]Inositol monophosphate formed was recovered by standard ion exchange chromatographic techniques and quantitated by liquid scintillation counting. Inverse agonist activity was determined by the degree to which a test compound reduced basal (cells incubated with vehicle) levels of [³H]inositol monophosphate accumulation.

### Abbreviations Used

The following abbreviations have the meanings indicated, unless stated otherwise in the specification: DIPEA=*N,N*-diisopropylethylamine; DMF=*N,N*-dimethylformamide; DMSO= dimethyl sulfoxide; EDC or EDCI=1-(3-dimethylaminopropyl)3-ethylcarbodiimide HCl; eq.= equivalent(s); ES (or ESI) - MS=electron spray ionization - mass spectroscopy; EtOAc=ethyl acetate; FAB-MS=fast atom bombardment-mass spectroscopy; HCl=hydrogen chloride; HOAt= 1-hydroxy-7-azabenzotriazole; HPLC=high pressure liquid chromatography; LCMS=liquid chromatography/mass spectroscopy; LAH=Lithium aluminum hydride; MeOH=methanol; MHz=megahertz; NMR=nuclear magnetic resonance; NOBF₄=nitrosonium tetrafluoroborate; RT=Room temperature; TEA₌triethylamine; TFA=trifluoroacetic acid; THF=tetrahydrofuran.

The general method of preparation of biarylmethanamine derivatives is depicted in Scheme 1. Reduction of the nitro group and nitrile hydrolysis of known pyridine (1) (J.Chem. Soc, (1952), 2042-2046.) is followed by conversion of the resultant amine to the fluoride to afford (2a). Alternatively (1) may be converted to chloride (2b) by a similar reduction/chlorination sequence. The amide (2a/2b) is converted in a 3 step sequence to aldehyde (3). Imine formation with chiral t-butyl sulfonamide (Tetrahedron (1999), 8883-8904) is followed by addition of methyl Grignard to produce (4). The bromide is converted to a boronate through use of a catalyst like palladium and reagent (5) and the resulting intermediate used in a Suzuki coupling with a halide partner such as (6). The resulting bi-aryl is then deprotected using an acid such as HCl to afford amine (7) which is acylated with acid (8) using a reagent such as EDC. Deprotection of the Boc group using HCl and acylation of the resultant amine with the appropriate carboxylic acid equivalent affords the target molecule (I).

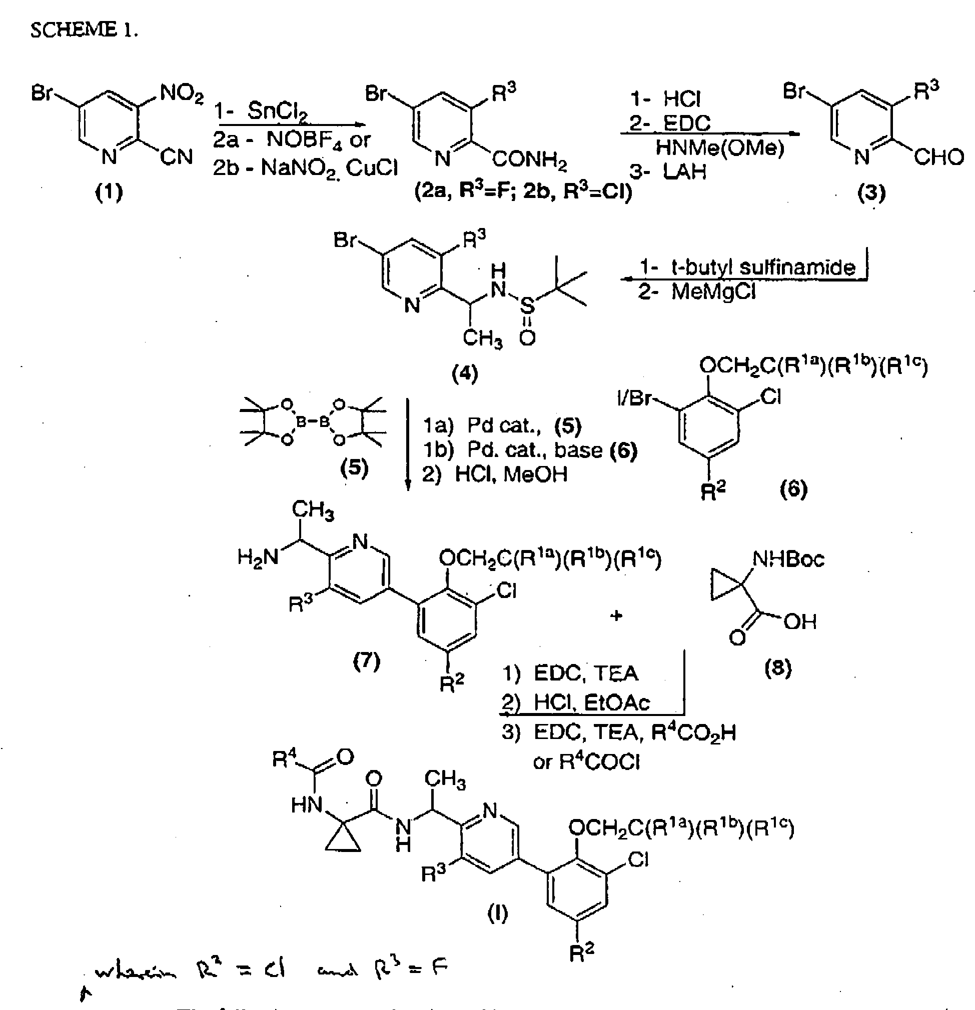

The following compounds are provided for illustrative purpose only and are not to be construed as limiting the scope of the invention in any manner.

### EXAMPLE 1

### N-(1-{[((1R)-1-{5-[3,5-dichloro-2-(2,2-difluoroethoxy)phenyl]-3-fluoropyridin-2-yl}ethyl)-amino]carbonyl}cyclopropyl)-5-methylisoxazole-3-carboxamide

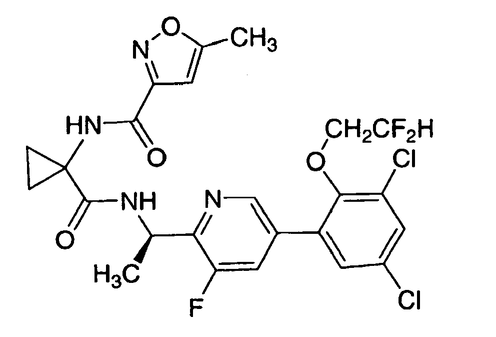

Step 1. To a solution of 5-bromo-3-nitropyridine-2-carbonitrile (4.71 g, 20.7 mmol) in MeOH (319 mL) under N₂ was added tin(II) chloride dihydrate (27.97 g, 123.9 mmol). The reaction mixture was heated to 40 °C for 40 minutes, concentrated in vacuo, and azeotroped with toluene. The residue was dissolved in EtOAc, and 10% aqueous sodium bicarbonate was added to render the reaction mixture basic. The aqueous layer was extracted with CHCl₃ 3x and the combined organics were dried over Na₂SO₄, filtered and concentrated under vacuum to provide 3-amino-5-bromopyridine-2-carboxamide that gave a mass ion (ES+) of 218.2 for M+H⁺(⁸¹Br).

Step 2. To a solution of the compound of Step 1 (40.0 g, 185.2 mmol) in CH₂Cl₂ was added nitrosonium tetrafluoroborate (22.71g, 191.4 mmol). The reaction mixture was stirred at RT for 4.5 hours, then concentrated in vacuo and azeotroped with toluene. The residue was suspended in toluene (1100 mL) and heated to 100 °C for 2 hours. The reaction mixture was concentrated in vacuo, and the residue suspended in CH₂Cl₂. The solid was collected to provide 5-bromo-3-fluoropyridine-2-carboxamide that gave proton NMR spectra consistent with theory and a mass ion (ES+) of 219.1 for M+H⁺(⁷⁹Br).

Step 3. A solution of crude compound of Step 2 (40.50 g, 184.9 mmol) in HCl (12N, 539.4 mL, 6.472 mol) was heated to 120 °C for 1 hour. The reaction mixture was cooled to RT, and aqueous NaOH (20%) was added slowly until the reaction mixture was approximately pH -6. The solution was concentrated in vacuo, and azeotroped with toluene. The residue was thoroughly extracted with 40% MeOH/CHCl₃ and filtered. This cycle was repeated three times. The combined filtrates were concentrated and azeotroped 3x with toluene to provide 5-bromo-3-fluoropyridine-2-carboxylic acid that gave a mass ion (ES+) of 218.1 for M+H⁺(⁷⁹Br).

Step 4. A solution of crude compound of Step 3 (40.65 g, 184.8 mmol), N,O-dimethylhydroxylamine HCl (21.63 g, 221.7 mmol), EDC (70.85 g, 369.6 mmol), HOAt (2.497 g, 18.48 mmol), and TEA (16.48 mL, 118.3 mmol) in 200 mL DMF was stirred at RT overnight. The solution was partially concentrated in vacuo and partitioned between EtOAc and 10% aqueous sodium bicarbonate. The aqueous layer was extracted with EtOAc 4x, and the combined organic extracts were washed with water and brine, dried over Na₂SO₄, filtered and concentrated to provide 5-bromo-3-fluoro-N-methoxy-N-methylpyridine-2-carboxamide that gave proton NMR spectra consistent with theory and a mass ion (ES+) of 263.01 for M+H+(79Br).

Step 5*.* To a solution of crude compound of Step 4 (27.94 g, 106.2 mmol) in THF (350 mL) at -78 °C was added LiAlH₄ (1M in THF, 45.67 mL, 45.67 mmol) dropwise. The reaction mixture was stirred at - 78 °C for 2 hours, then H₂O (100 mL) and brine (100 mL) were added. The mixture was warmed to RT and partially concentrated in vacuo, diluted with EtOAc and filtered through celite. The aqueous layer was extracted with EtOAc 4x. The combined organic extracts were washed with brine, dried over Na₂SO₄, filtered and concentrated. The residue was filtered through a silica gel plug with 10% EtOAc in hexanes to provide 5-bromo-3-fluoropyridine-2-carbaldehyde that gave proton NMR spectra consistent with theory.

Step 6. To a solution of (R)-(+)-2-methyl-2-propanesulfinamide (9.898 g, 81.67 mmol) in CH₂Cl₂ (160 mL) was added the compound of Step 5 (16.66 g, 81.67 mmol), pyridinium p-toluenesulfonate (1.026 g, 4.08 mmol), and magnesium sulfate (49.15 g, 408.3 mmol). The reaction mixture was stirred at RT overnight, then filtered through celite and concentrated in vacuo. The residue was subjected to silica gel chromatography and eluted with 0 to 10% EtOAc in hexanes to provide N-[(1E)-(5-bromo-3-fluoro-pyridin-2-yl)methylidene]-2-methylpropane-2-sulfinamide that gave proton NMR spectra consistent with theory and a mass ion (ES+) of 307.0 for M+H⁺ (⁸¹Br).

Step 7. A solution of the compound of Step 6 (18.63 g, 60.65 mmol) in CH₂Cl₂ (375 mL) was cooled to -50°C under N₂. Methylmagnesium chloride (3M in THF, 30.32 mL, 90.97 mmol) was added dropwise and the reaction mixture was stirred for 1h. Additional methylmagnesium chloride (5.0 mL, 15.0 mmol) was added after 30 minutes to drive the reaction to completion. Water (200 mL) and brine (200 mL) were added, and the reaction mixture was allowed to warm to RT. The aqueous layer was extracted with CH₂Cl₂ 4x, and the combined organic extracts were dried over Na₂SO₄, filtered and concentrated under vacuum. The residue was subjected to silica gel chromatography and eluted with 10-40% EtOAc in hexanes to provide N-[(1R)-1-(5-bromo-3-fluoropyridin-2-yl)ethyl]-2-methylpropane-2-sulfinamide that gave proton NMR spectra consistent with theory and a mass ion (ES+) of 325.0 for M+H⁺ (81_{Br}).

Step 8. A mixture of 2-bromo-4,6-dichlorophenol (9.50 g, 39.27 mmol), 1,1-difluoro-2-iodoethane (10.93 g, 56.95 mmol) and potassium carbonate (10.86 g, 78.55 mmol) were suspended in 25 mL DMF, and heated to 100 °C in a 350 mL pressure vessel for 6 hours. The suspension was cooled to RT, and partitioned between ethyl ether and water. The organic extracts were washed with water and brine, dried over MgSO₄, filtered and concentrated under vacuum. The residue was subjected to silica gel chromatography and eluted with hexanes to provide 1-bromo-3,5-dichloro-2-(2,2-diflurorethoxy)benzene that gave proton NMR spectra consistent with theory.

Step 9. A mixture of the compound of Step 7 (10.70 g, 33.10 mmol), bis(pinacolato)diboron (12.61 g, 49.66 mmol), potassium acetate (9.75 g, 99.31 mmol), and [1,1'-bis(diphenylphosphino)ferrocene]-palladium(II) dichloride (PdCl₂(dppf), 2.42 g, 3.31 mmol) in 45 mL DMSO was purged with N₂ and was heated to 80 °C under N₂ in a pressure vessel for 24 hours. The reaction mixture was cooled to RT, and the compound of Step 8 (15.19 g, 49.66 mmol), potassium carbonate (11.44 g, 82.76 mmol), water (8 mL), and PdCl₂(dppf) (2.42 g, 3.31 mmol) were added. The reaction mixture was heated to 80 °C for 21 hours, then cooled to RT and partitioned between ethyl ether and water. The organic extract was washed with water and brine, dried over Na₂SO₄, filtered and concentrated under vacuum. The residue was subjected to silica gel chromatography and eluted with 0-35% EtOAc in hexanes to provide N-((1R)-1-{5-[3,5-dichloro-2-(2,2-difluoroethoxy)phenyl]-3-nuoropyridin-2-yl}ethyl)-2-methylpropane-2-sulfinamide that gave a mass ion (ES+) of 469.0 for M+H⁺.

Step 10. To a solution of the above reaction product (1.40 g, 2.98 mmol) in methanol (5 mL) at 0 °C, was added a HCl/dioxane solution (4M, 2.98 mL, 11.93 mmol). The resulting reaction mixture was stirred at 0 °C for 20 minutes. The reaction mixture was concentrated in vacuo to provide 2-[(1R)-1-ammonioethyl-5-[3,5-dichloro-2-(2,2-difluoroethoxy)phenyl]-3-fluoropyridinium dichloride that gave a mass ion (ES+) of 365.1 for M+H⁺.

Step 11. A solution of t-butoxycarbonyl-1-aminocyclopropane-1-carboxylic acid (0.903 g, 4.49 mmol), EDC (1.03 g, 5.38 mmol), and HOAt (0.407 g, 2.99 mmol) in 4 mL CH₂Cl₂ was stirred at RT for 10 minutes. The solution was added to the compound of Step 10 (1.31 g, 2.99 mmol), stirred at 0 °C and DIPEA was added to adjust the pH of the reaction mixture to 8. The reaction mixture was then stirred at RT for another 20 minutes and partitioned between EtOAc and water. The organic extracts were washed with water and brine, dried over Na₂SO₄, filtered and concentrated under vacuum. The residue was subjected to silica gel chromatography eluted with 0-40% EtOAc in hexanes to provide tert-butyl 1-{[((1R)-1-{5-[3,5-dichloro-2-(2,2-difluoroethoxy)phenyl]-3-fluoropyridin-2-yl}ethyl)amino]carbonyl}cyclopropyl carbamate that gave a mass ion (ES+) of 548.2 for M+H⁺.

Step 12. A solution of the above product (1.3 g, 2.37 mmol) in EtOAc (100 mL) was cooled in an ice bath, and then saturated with anhydrous hydrogen chloride gas. The solution stirred at 0 °C for 30 minutes more, and concentrated in vacuo to yield 2-((1R)-1-{[(1-ammoniocyclopropyl)-carbonyl]amino}ethyl-5-[3,5-dichloro-2-(2,2-difluoroethoxy)phenyl]-3-fluoropyridinium dichloride that gave a mass ion (ES+) of 448.1 for M+H⁺.

Step 13. To a solution of the compound of Step 12 (2.09 g, 4.01 mmol) in CH₂Cl₂ (12 mL) at 0 °C was added 5-methylisoxazole-3-carbonyl chloride (0.875 g, 6.02 mmol). DIPEA was added to adjust pH of the reaction mixture to 9. The reaction mixture was stirred at 0 °C for another 20 minutes and partitioned between EtOAc and water. The organic extract was washed with water and brine, dried over Na₂SO₄, filtered and concentrated under vacuum. The residue was subjected to silica gel chromatography and eluted with 0-50% EtOAc in hexanes to provide the title compound that gave proton NMR spectra consistent with theory and a mass ion of 557.08 for M+H⁺. ¹H NMR (400 MHz, DMSO-d₆) δ 9.23 (s, 1H), 8.48 (s, 1H), 8.11-8.09 (d, *J* = 7.7 Hz, 1H), 7.94-7.91 (m, 1H), 7.81-7.80 (d, *J* = 2.6 Hz, 1H), 7.63-7.62 (d, *J* = 2.6 Hz, 1H), 6.56 (s, 1H), 6.15-5.87 (m, 1H). 5.34-5.27 (m, 1H), 3.99-3.90 (m, 2H), 2.46 (s, 3H), 1.40-1.28 (m, 5H), 1.08-0.97 (m, 2H).

### EXAMPLE 2

### N-(1-{[((1R)-1-{5-[3,5-dichloro-2-(2,2-difluoroethoxy)phenyl]-3-fluoropyridin-2-yl}lethyl)-aminol]carbonyl}cyclopropyl)-3-methoxyisoxazole-5-carboxamide

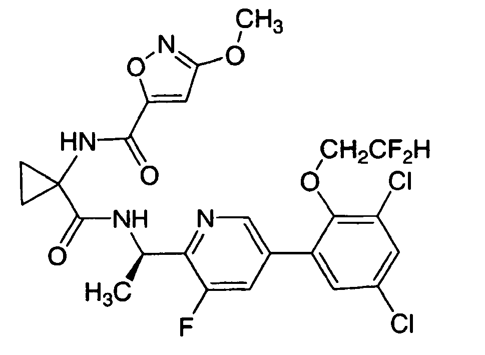

A solution of 3-methoxyisoxazole-5-carboxylic acid (0.240 g, 1.68 mmol), EDC (0.257 g, 1.34 mmol), and HOAt (0.073 g, 0.54 mmol) in 1 mL DMF was stirred at RT for 10 minutes. The solution was added to the compound of Example 1, Step 12 (0.35 g, 0.67 mmol), and sufficient DIPEA was added to adjust pH of the reaction mixture to 8. The reaction mixture was stirred at RT for 30 minutes and partitioned between EtOAc and water. The organic extracts were washed with water and brine, dried over Na₂SO₄, filtered and concentrated under vacuum. The residue was subjected to silica gel chromatography and eluted with 0-40% EtOAc in hexanes to provide the title compound that gave proton NMR spectra consistent with theory and a mass ion of 573.09 for M+H⁺. ¹H NMR (400 MHz, DMSO-d₆) δ 9.32 (s, 1H), 8.51 (s, 1H), 8.30-8.28 (d, *J* = 7.7 Hz, 1H), 7.92-7.89 (m, 1H), 7.81-7.80 (d, *J* = 2.6 Hz, 1H), 7.63-7.62 (d, *J* = 2.6 Hz, 1H), 6.82 (s, 1H), 6.16-5.87 (m, 1H), 5.35-5.31 (m, 1H), 3.94 (s, 3H), 3.99-3.90 (m, 2H), 1.42-1.24 (m, 5H), 1.07-0.95 (m, 2H).

### EXAMPLE 3

### N-(1-{[((1R)-1-{5-[3,5-dichloro-2-(2,2-difluoroethoxy)phenyl]-3-fluoropyridin-2-yl}ethyl)amino] carbonyl}cyclopropyl)-5-(trifluoromethyl)-2-furamide

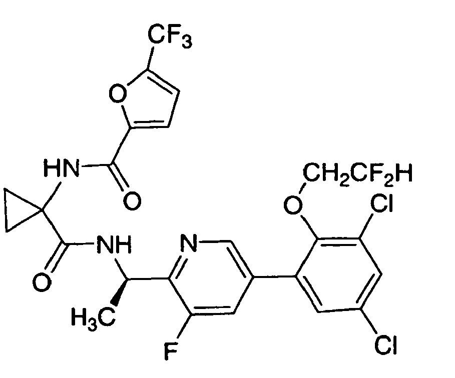

A solution of 5-trifluoromethyl-2-furoic acid (0.054 g, 0.3 mmol), EDC (0.038 g, 0.2 mmol), and HOAt (0.014 g, 0.1 mmol) in CH₂Cl₂ (1 mL) was stirred at RT for 10 minutes. The solution was added to the compound of Example 1, Step 12 (0.053 g, 0.1 mmol), and sufficient DIPEA was added to adjust pH of the reaction mixture to 9. The reaction mixture was stirred at RT for 30 minutes and subjected to silica gel chromatography and eluted with 0-40% EtOAc in hexanes to provide the title compound that gave proton NMR spectra consistent with theory and a mass ion of 610.07 for M+H⁺.
¹H NMR (400 MHz, DMSO-d₆) δ 9.19 (s, 1H), 8.49 (s, 1H), 8.26-8.24 (d, *J* = 7.6 Hz, 1H), 7.92-7.89 (m, 1H), 7.81-7.80 (d, *J* = 2.6 Hz, 1H), 7.62-7.61 (d, J = 2.6 Hz, 1H), 7.39-7.38 (d, *J* = 3.7 Hz, 1H), 7.33-7.32 (d, *J* = 3.7 Hz, 1H), 6.15-5.87 (m, 1H), 5.35-5.32 (m, 1H), 3.98-3.90 (m, 2H), 1.40-1.23 (m, 5H), 1.07-0.96 (m, 2H).

### EXAMPLE 4

### N-(1-{[((1R)-1-{5-[3,5-dichloro-2-(2,2-difluoroethoxy)phenyl]-3-fluoropyridin-2-yl}ethyl)amino]-carbonyl}cyclopropyl)pyrazine-2-carboxamide

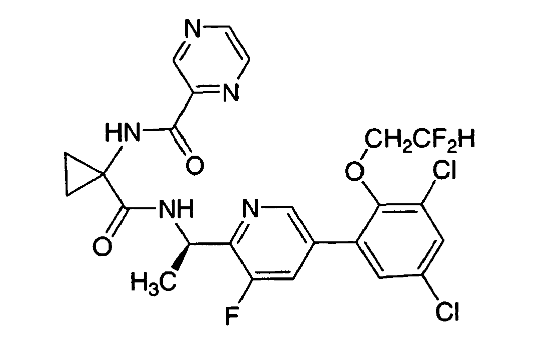

A solution of of the compound of Example 1, Step 12 (0.041 g, 0.085 mmol), pyrazine-2-carboxylic acid (0.0158 g, 0.13 mmol), (1*H*-1,2,3-benzotriazol-1-yloxy)[tris(dimethylamino)]-phosphonium hexafluorophosphate (0.0752 g, 0.17 mmol), and TEA (0.04 mL, 0.26 mmol) in 1.5 mL of DMF was stirred at RT for 1 hour. The solution was diluted with EtOAc, and washed three times with water. The organic layer is dried over Na₂SO₄, filtered and concentrated to an oil. The residue was subjected to silica gel chromatography and eluted with 0-40% EtOAc in hexanes to afford the title compound that gave proton NMR spectra consistent with theory and a mass ion (ES+) of 554.0 for M+H⁺. ¹H NMR CD₃OD δ 1.21 (m, 2H), 9.25 (s, 1H), 8.81 (d, J = 2.4 Hz, 1H), 8.72 (d, J = 1.5Hz, 1H), 8.37 (s, 1H), 8.28 (d, J = 7.6 Hz, 1H), 7.78 (d, J = 10.7 Hz, 1H), 7.60 (d, J = 2.6 Hz, 1H), 7.44 (d, J = 2.5 Hz, 1H), 5.83 (t, J = 54.6, 54.6 Hz, 1H), ), 5.46 (m, 1H), 3.90 (m, 2H), 3.90 (m, 2H), 1.46 (d, J = 6.9 Hz, 3H), 1.21 (m, 2H).

The following compounds were prepared following the procedures provided in the above examples and the general scheme. The starting materials are either commercially available or may be prepared from commercially available reagents using conventional reactions well known in the art.

**Table 1**

| | | | | | |
|---|---|---|---|---|---|
| 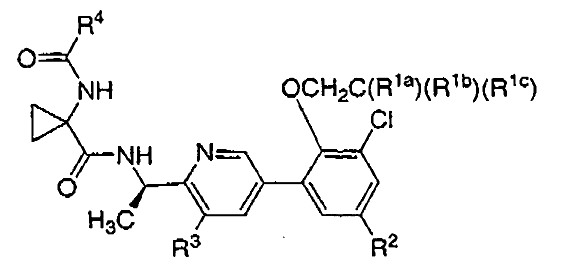 | | | | | |

| Example | R⁴ | C(R^{1a})(R^{1b})(R^{1c}) | R² | R³ | M+H⁺ |
|---|---|---|---|---|---|
| 5 | 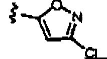 | CF₂H | Cl | F | 577 |
| 7 | 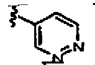 | CH₃ | Cl | F | 518 |
| 8 | 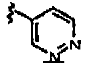 | CF₂H | Cl | F | 554 |
| 10 | 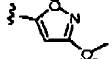 | CF₃ | Cl | F | 591 |
| 11 | 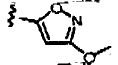 | CH₃ | Cl | F | 537 |
| 12 | 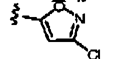 | CH₃ | Cl | F | 541 |
| 13 | 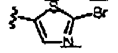 | CH₃ | Cl | F | 603 |
| 15 | 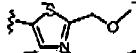 | CH₃ | Cl | F | 567 |
| 16 | 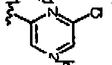 | CF₂H | Cl | F | 590 |
| 17 | 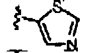 | CH₃ | Cl | F | 523 |
| 18 | 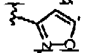 | CH₃ | Cl | F | 507 |
| 19 | 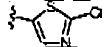 | CH₃ | Cl | F | 557 |
| 21 | 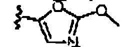 | CH₃ | Cl | F | 537 |
| 22 | 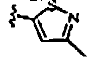 | CH₃ | Cl | F | 537 |
| 25 | 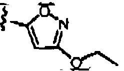 | CF₂H | Cl | F | 587 |
| 26 | 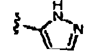 | CH₃ | Cl | F | 506 |
| 27 | 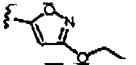 | CH₃ | Cl | F | 551 |
| 28 | 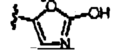 | CH₃ | Cl | F | 559 |
| 30 | 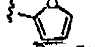 | CH₃ | Cl | F | 506 |
| 31 | 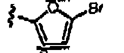 | CH₃ | Cl | F | 584 |
| 32 | 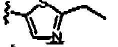 | CH₃ | Cl | F | 551 |
| 33 | 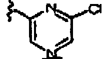 | CH₃ | Cl | F | 552 |
| 34 | 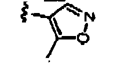 | CH₃ | Cl | F | 521 |
| 35 | 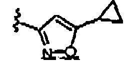 | CH₃ | Cl | F | 547 |
| 37 | 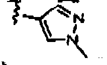 | CF₂H | Cl | F | 556 |
| 38 | 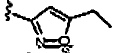 | CF₂H | Cl | F | 571 |
| 39 | 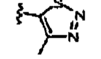 | CH₃ | Cl | F | 538 |
| 41 | 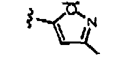 | CH₃ | Cl | F | 521 |
| 42 | 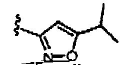 | CF₂H | Cl | F | 585 |
| 43 | 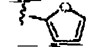 | CF₂H | Cl | F | 542 |
| 44 | 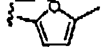 | CF₂H | Cl | F | 556 |
| 45 | 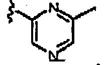 | CH₃ | Cl | F | 532 |
| 46 | 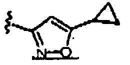 | CF₂H | Cl | F | 583 |
| 48 | 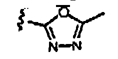 | CH₃ | Cl | F | 522 |
| 49 | 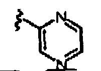 | CH₃ | Cl | F | 518 |
| 50 | 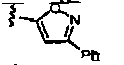 | CF₂H | Cl | F | 619 |
| 52 | 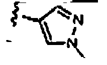 | CH₃ | Cl | F | 520 |
| 54 | 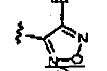 | CF₂H | Cl | F | 558 |
| 55 | 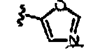 | CH₃ | Cl | F | 507 |
| 57 | 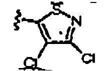 | CH₃ | Cl | F | 593 |
| 58 | 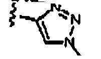 | CF₂H | Cl | F | 557 |
| 59 | 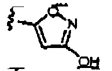 | CF₂H | Cl | F | 559 |
| 61 | 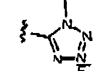 | CH₃ | Cl | F | 522 |
| 63 | 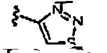 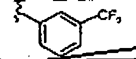 | CH₃ | Cl | F | 524 |
| 70 | 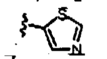 | CF₂H | Cl | F | 559 |
| 71 | 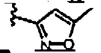 | CH₃ | Cl | F | 521 |
| 73 | 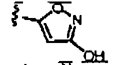 | CH₃ | Cl | F | 523 |
| 74 | 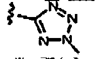 | CH₃ | Cl | F | 522 |
| 75 | 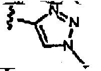 | CH₃ | Cl | F | 521 |
| 77 | 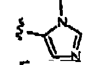 | CH₃ | Cl | F | 520 |
| 78 | 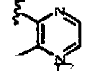 | CH₃ | Cl | F | 532 |
| 79 | 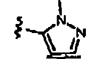 | CH₃ | Cl | F | 520 |
| 80 | 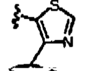 | CH₃ | Cl | F | 537 |
| 81 | 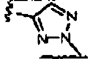 | CH₃ | Cl | F | 521 |
| 82 | 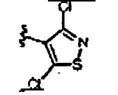 | CH₃ | Cl | F | 593 |
| 84 | 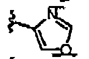 | CH₃ | Cl | F | 507 |
| 85 | 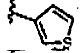 | CH₃ | Cl | F | 522 |
| 86 | 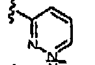 | CH₃ | Cl | F | 518 |
| 87 | 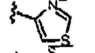 | CH₃ | Cl | F | 523 |
| 88 | 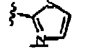 | CH₃ | Cl | F | 523 |
| 89 | 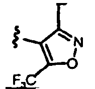 | CF₂H | Cl | F | 625 |
| 90 | 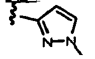 | CH₃ | Cl | F | 520 |
| 91 | 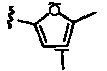 | CH₃ | Cl | F | 534 |
| 92 | 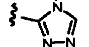 | CH₃ | Cl | F | 521 |

## Claims

1. A compound having the formula la and pharmaceutically acceptably salts thereof: 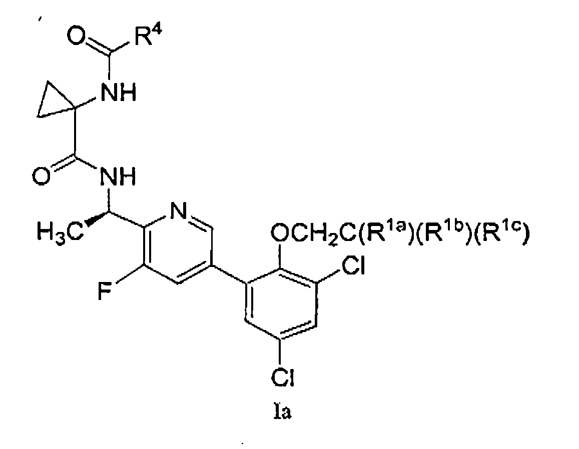 wherein R^{1a}, R^{1b} and R^{1c} are each independently selected from hydrogen and fluorine; R⁴ is (a) optionally substituted 5-membered heteroaromatic ring having a ring heteroatom selected from N, O and S, and optionally having up to 3 additional ring nitrogen atoms; or (b) optionally substituted 6-membered heteroaromatic ring containing from 1 to 3 ring nitrogen atoms and N-oxides thereof; wherein the substitutent is 1 to 2 groups independently selected from halogen, C₁₋₄alkyl optionally substituted with C₁₋₄alkoxy, C₁₋₄alkoxy, hydroxy, C₃₋₆ cycloalkyl, and CF₃.

2. A compound of Claim 1 wherein R⁴ is selected from optionally substituted isoxazolyl, optionally substituted oxazolyl, optionally substituted isothiazolyl, optionally substituted thiazolyl, optionally substituted pyridazinyl and optionally substituted pyrazinyl, wherein the substituent is 1 to 2 groups selected from halogen, C₁₋₄alkyl optionally substituted with C₁₋₄alkoxy, C₁₋₄alkoxy, hydroxy, and CF₃.

3. A compound of Claim 1 wherein R⁴ is selected from 3-chloro-5-isoxazolyl, 3-methoxy-5-isoxazolyl, 3-ethoxy-5-isoxazolyl, and 3-methyl-5-isoxazolyl.

4. A compound of Claim 1 selected from: 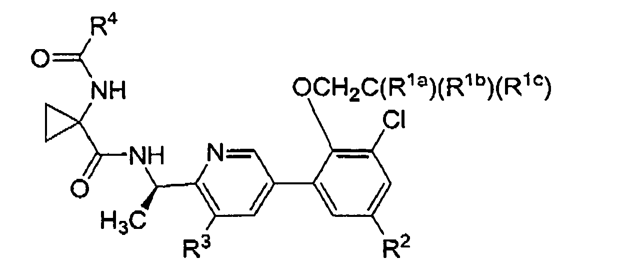
| R⁴ | C(R^{1a})(R^{1b})(R^{1c}) | R² | R³ |
|---|---|---|---|
| 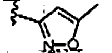 | CF₂H | Cl | F |
| 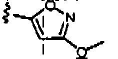 | CF₂H | Cl | F |
| 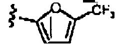 | CF₂H | Cl | F |
| 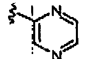 | CF2H | Cl | F |
| 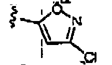 | CF₂H | Cl | F |
| 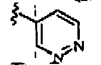 | CH₃ | Cl | F |
| 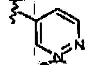 | CF₂H | Cl | F |
| 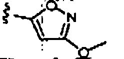 | CF₃ | Cl | F |
| 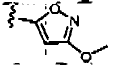 | CH₃ | Cl | F |
| 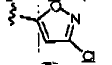 | CH₃ | Cl | F |
| 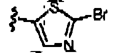 | CH₃ | Cl | F |
| 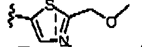 | CH₃ | Cl | F |
| 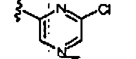 | CF₂H | Cl | F |
| 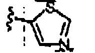 | CH₃ | Cl | F |
| 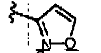 | CH₃ | Cl | F |
| 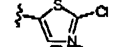 | CH₃ | Cl | F |
| 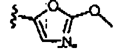 | CH₃ | Cl | F |
| 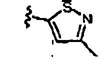 | CH₃ | Cl | F |
| 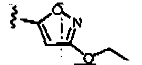 | CF₂H | Cl | F |
| 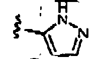 | CH₃ | Cl | F |
| 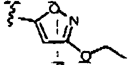 | CH₃ | Cl | F |
| 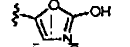 | CH₃ | Cl | F |
| 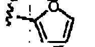 | CH₃ | Cl | F |
| 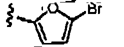 | CH₃ | Cl | F |
| 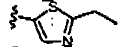 | CH₃ | Cl | F |
| 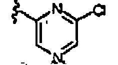 | CH₃ | Cl | F |
| 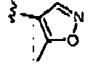 | CH₃ | Cl | F |
| 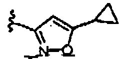 | CH₃ | Cl | F |
| 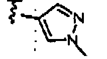 | CF₂H | Cl | F |
| 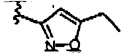 | CF₂H | Cl | F |
| 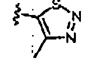 | CH₃ | Cl | F |
| 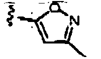 | CH₃ | Cl | F |
| 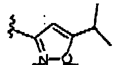 | CF₂H | Cl | F |
| 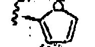 | CF₂H | Cl | F |
| 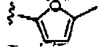 | CF₂H | Cl | F |
| 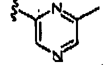 | CH₃ | Cl | F |
| 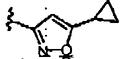 | CF₂H | Cl | F |
| 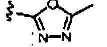 | CH₃ | Cl | F |
| 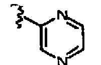 | CH₃ | Cl | F |
| 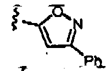 | CF₂H | Cl | F |
| 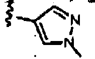 | CH₃ | Cl | F |
| 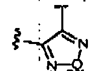 | CF₂H | Cl | F |
| 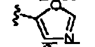 | CH₃ | Cl | F |
| 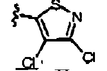 | CH₃ | Cl | F |
| 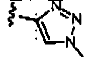 | CF₂H | Cl | F |
| 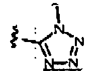 | CH₃ | Cl | F |
| 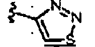 | CH₃ | Cl | F |
| 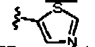 | CF₂H | Cl | F |
| 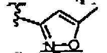 | CH₃ | Cl | F |
| 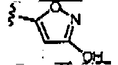 | CH₃ | Cl | F |
| 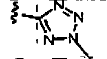 | CH₃ | Cl | F |
| 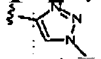 | CH₃ | Cl | F |
| 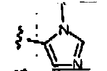 | CH₃ | Cl | F |
| 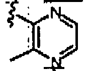 | CH₃ | Cl | F |
| 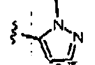 | CH₃ | Cl | F |
| 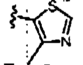 | CH₃ | Cl | F |
| 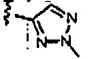 | CH₃ | Cl | F |
| 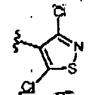 | CH₃ | Cl | F |
| 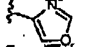 | CH₃ | Cl | F |
| 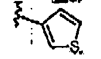 | CH₃ | Cl | F |
| 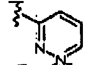 | CH₃ | Cl | F |
| 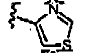 | CH₃ | Cl | F |
| 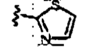 | CH₃ | Cl | F |
| 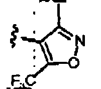 | CF₂H | Cl | F |
| 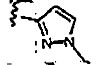 | CH₃ | Cl | F |
| 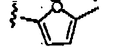 | CH₃ | Cl | F |
| 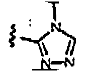 | CH₃ | Cl | F |
and pharmaceutically acceptable salts thereof.

5. A pharmaceutical composition which comprises a therapeutically effective amount of a compound of any of claims 1-4 or a pharmaceutically acceptable salt thereof and a pharmaceutically acceptable carrier.

6. Use of a compound of any of claims 1-4 or a pharmaceutically acceptable salt thereof for the manufacture of a medicament for the treatment of conditions mediated by bradykinin B1 receptor.

## Patentansprüche

1. Eine Verbindung mit der Formel Ia und pharmazeutisch annehmbare Salze davon: 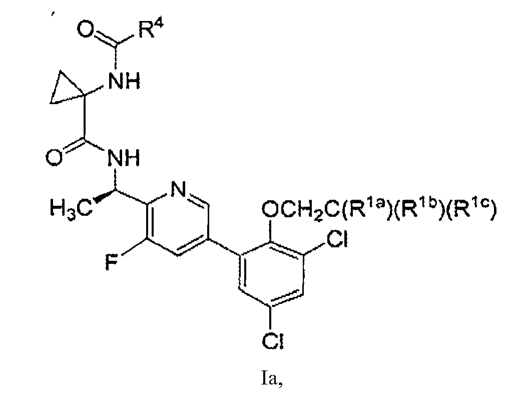 wobei R^{1a}, R^{1b} und R^{1c} jeweils unabhängig ausgewählt sind aus Wasserstoff und Fluor, R⁴ (a) gegebenenfalls substituierter 5-gliedriger heteroaromatischer Ring mit einem Ring-Heteroatom, ausgewählt aus N, O und S, und gegebenenfalls mit bis zu 3 zusätzlichen Ring-Stickstoffatomen; oder (b) gegebenenfalls substituierter 6-gliedriger heteroaromatischer Ring, der 1 bis 3 Ring-Stickstoffatome und N-Oxide davon enthält, ist; wobei der Substituent 1 bis 2 Gruppen ist, unabhängig ausgewählt aus Halogen, C₁₋₄-Alkyl, gegebenenfalls substituiert mit C₁₋₄-Alkoxy, C₁₋₄-Alkoxy, Hydroxy, C₃₋₆-Cycloalkyl und CF₃.

2. Eine Verbindung nach Anspruch 1, wobei R⁴ ausgewählt ist aus gegebenenfalls substituiertem Isoxazolyl, gegebenenfalls substituiertem Oxazolyl, gegebenenfalls substituiertem Isothiazolyl, gegebenenfalls substituiertem Thiazolyl, gegebenenfalls substituiertem Pyridazinyl und gegebenenfalls substituiertem Pyrazinyl, wobei der Substituent 1 bis 2 Gruppen ist, ausgewählt aus Halogen, C₁₋₄-Alkyl, gegebenenfalls substituiert mit C₁₋₄-Alkoxy, C₁₋₄-Alkoxy, Hydroxy und CF₃.

3. Eine Verbindung nach Anspruch 1, wobei R⁴ ausgewählt ist aus 3-Chlor-5-isoxazolyl, 3-Methoxy-5-isoxazolyl, 3-Ethoxy-5-isoxazolyl und 3-Methyl-5-isoxazolyl.

4. Eine Verbindung nach Anspruch 1, ausgewählt aus: 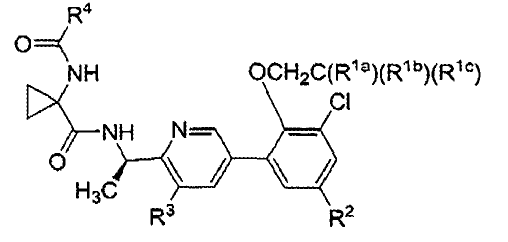
| R⁴ | C(R^{1a})(R^{1b})(R^{1c}) | R² | R³ |
|---|---|---|---|
| 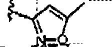 | CF₂H | Cl | F |
| 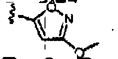 | CF₂H | Cl | F |
| 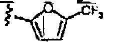 | CF₂H | Cl | F |
| 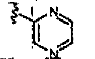 | CF2H | Cl | F |
| 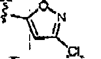 | CF₂H | Cl | F |
| 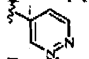 | CH₃ | Cl | F |
| 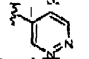 | CF₂H | Cl | F |
| 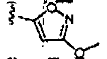 | CF₃ | Cl | F |
| 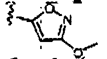 | CH₃ | Cl | F |
| 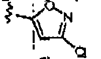 | CH₃ | Cl | F |
| 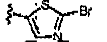 | CH₃ | Cl | F |
| 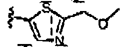 | CH₃ | Cl | F |
| 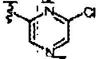 | CF₂H | Cl | F |
| 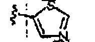 | CH₃ | Cl | F |
| 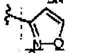 | CH₃ | Cl | F |
| 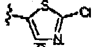 | CH₃ | Cl | F |
| 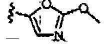 | CH₃ | Cl | F |
| 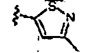 | CH₃ | Cl | F |
| 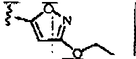 | CF₂H | Cl | F |
| 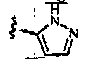 | CH₃ | Cl | F |
| 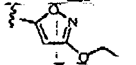 | CH₃ | Cl | F |
| 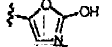 | CH₃ | Cl | F |
| 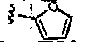 | CH₃ | Cl | F |
| 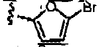 | CH₃ | Cl | F |
| 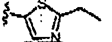 | CH₃ | Cl | F |
| 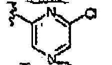 | CH₃ | Cl | F |
| 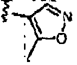 | CH₃ | Cl | F |
| 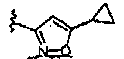 | CH₃ | Cl | F |
| 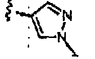 | CF₂H | Cl | F |
| 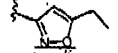 | CF₂H | Cl | F |
| 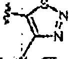 | CH₃ | Cl | F |
| 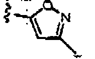 | CH₃ | Cl | F |
| 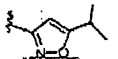 | CF₂H | Cl | F |
| 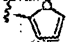 | CF₂H | Cl | F |
| 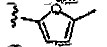 | CF₂H | Cl | F |
| 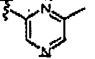 | CH₃ | Cl | F |
| 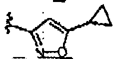 | CF₂H | Cl | F |
| 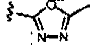 | CH₃ | Cl | F |
| 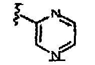 | CH₃ | Cl | F |
| 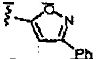 | CF₂H | Cl | F |
| 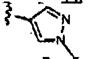 | CH₃ | Cl | F |
| 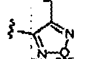 | CF₂H | Cl | F |
| 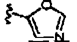 | CH₃ | Cl | F |
| 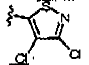 | CH₃ | Cl | F |
| 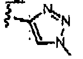 | CF₂H | Cl | F |
| 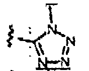 | CH₃ | Cl | F |
| 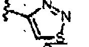 | CH₃ | Cl | F |
| 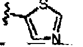 | CF₂H | Cl | F |
| 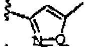 | CH₃ | Cl | F |
| 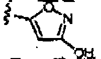 | CH₃ | Cl | F |
| 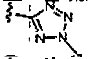 | CH₃ | Cl | F |
| 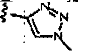 | CH₃ | Cl | F |
| 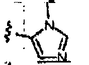 | CH₃ | Cl | F |
| 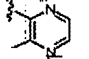 | CH₃ | Cl | F |
| 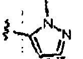 | CH₃ | Cl | F |
| 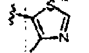 | CH₃ | Cl | F |
| 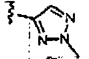 | CH₃ | Cl | F |
| 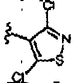 | CH₃ | Cl | F |
| 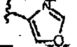 | CH₃ | Cl | F |
| 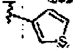 | CH₃ | Cl | F |
| 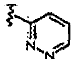 | CH₃ | Cl | F |
| 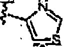 | CH₃ | Cl | F |
| 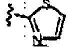 | CH₃ | Cl | F |
| 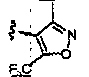 | CF₂H | Cl | F |
| 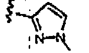 | CH₃ | Cl | F |
| 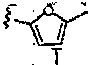 | CH₃ | Cl | F |
| 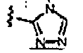 | CH₃ | Cl | F |
und pharmazeutisch annehmbaren Salzen davon.

5. Eine pharmazeutische Zusammensetzung, die eine therapeutisch wirksame Menge einer Verbindung nach irgendeinem der Ansprüche 1 - 4 oder eines pharmazeutisch annehmbaren Salzes davon und einen pharmazeutisch annehmbaren Träger enthält.

6. Verwendung einer Verbindung nach irgendeinem der Ansprüche 1 - 4 oder eines pharmazeutisch annehmbaren Salzes davon zur Herstellung eines Medikaments zur Behandlung von Zuständen, die durch Bradykinin-B1-Rezeptor vermittelt werden.

## Revendications

1. Composé présentant la formule Ia et sels pharmaceutiquement acceptables de celui-ci: 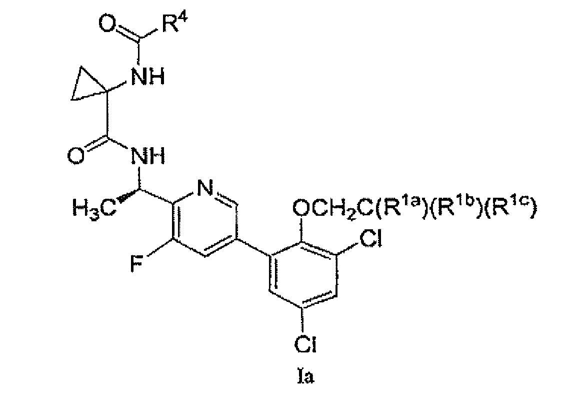 dans lequel R^{1a}, R^{1b} et R^{1c} sont chacun indépendamment choisis parmi un hydrogène et un fluor;
R⁴ est (a) un cycle hétéroaromatique à 5 membres éventuellement substitué possédant un hétéroatome de cycle choisi parmi N, O et S et possédant éventuellement jusqu'à 3 atomes d'azote supplémentaires; ou (b) un cycle hétéroaromatique à 6 membres éventuellement substitué contenant de 1 à 3 atomes d'azote de cycle et des N-oxydes de celui-ci; où le substituant est de 1 à 2 groupes indépendamment choisis parmi halogène, alkyle C₁₋₄ éventuellement substitué par alcoxy C₁₋₄, alcoxy C₁₋₄, hydroxy, cycloalkyle C₃₋₆ et CF₃.

2. Composé selon la revendication 1, dans lequel R⁴ est choisi parmi des groupes isoxazolyle éventuellement substitué, oxazolyle éventuellement substitué, isothiazolyle éventuellement substitué, thiazolyle éventuellement substitué, pyridazinyle éventuellement substitué et pyrazinyle éventuellement substitué, où le substituant est de 1 à 2 groupes choisis parmi halogène, alkyle C₁₋₄ éventuellement substitué par alcoxy C₁₋₄, alcoxy C₁₋₄, hydroxy et CF₃.

3. Composé selon la revendication 1, dans lequel R⁴ est choisi parmi des groupes 3-chloro-5-isoxazolyle, 3-méthoxy-5-isoxazolyle, 3-éthoxy-5-isoxazolyle et 3-méthyl-5-isoxazolyle.

4. Composé selon la revendication 1, choisi parmi: 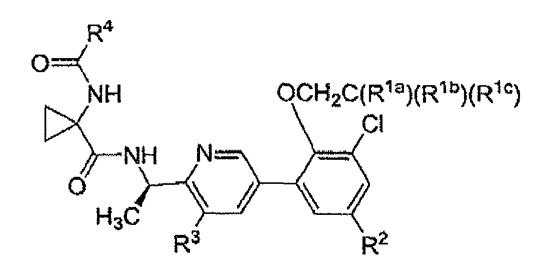
| R⁴ | C(R^{1a})(R^{1b})(R^{1c}) | R² | R³ |
|---|---|---|---|
| 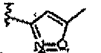 | CF₂H | Cl | F |
| 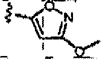 | CF₂H | Cl | F |
| 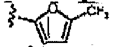 | CF₂H | Cl | F |
| 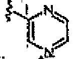 | CF₂H | Cl | F |
| 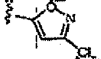 | CF₂H | Cl | F |
| 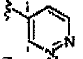 | CH₃ | Cl | F |
| 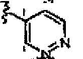 | CF₂H | Cl | F |
| 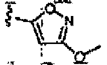 | CF₃ | Cl | F |
| 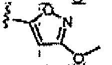 | CH₃ | Cl | F |
| 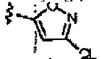 | CH₃ | Cl | F |
| 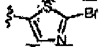 | CH₃ | Cl | F |
| 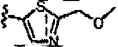 | CH₃ | Cl | F |
| 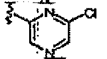 | CF₂H | Cl | F |
| 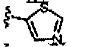 | CH₃ | Cl | F |
| 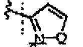 | CH₃ | Cl | F |
| 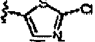 | CH₃ | Cl | F |
| 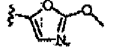 | CH₃ | Cl | F |
| 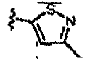 | CH₃ | Cl | F |
| 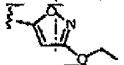 | CF₂H | Cl | F |
| 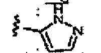 | CH₃ | Cl | F |
| 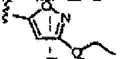 | CH₃ | Cl | F |
| 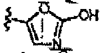 | CH₃ | Cl | F |
| 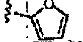 | CH₃ | Cl | F |
| 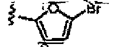 | CH₃ | Cl | F |
| 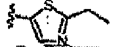 | CH₃ | Cl | F |
| 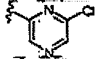 | CH₃ | Cl | F |
| 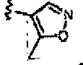 | CH₃ | Cl | F |
| 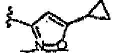 | CH₃ | Cl | F |
| 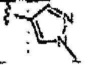 | CF₂H | Cl | F |
| 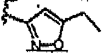 | CF₂H | Cl | F |
| 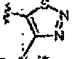 | CH₃ | Cl | F |
| 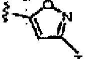 | CH₃ | Cl | F |
| 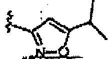 | CF₂H | Cl | F |
| 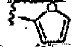 | CF₂H | Cl | F |
| 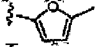 | CF₂H | Cl | F |
| 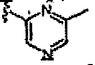 | CH₃ | Cl | F |
| 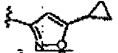 | CF₂H | Cl | F |
| 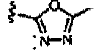 | CH₃ | Cl | F |
| 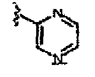 | CH₃ | Cl | F |
| 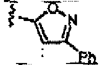 | CF₂H | Cl | F |
| 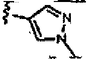 | CH₃ | Cl | F |
| 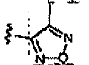 | CF₂H | Cl | F |
| 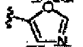 | CH₃ | Cl | F |
| 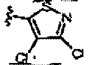 | CH₃ | Cl | F |
| 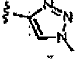 | CF₂H | Cl | F |
| 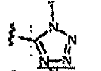 | CH₃ | Cl | F |
| 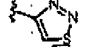 | CH₃ | Cl | F |
| 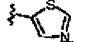 | CF₂H | Cl | F |
| 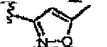 | CH₃ | Cl | F |
| 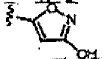 | CH₃ | Cl | F |
| 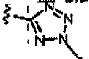 | CH₃ | Cl | F |
| 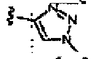 | CH₃ | Cl | F |
| 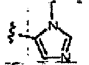 | CH₃ | Cl | F |
| 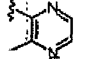 | CH₃ | Cl | F |
| 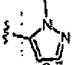 | CH₃ | Cl | F |
| 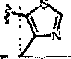 | CH₃ | Cl | F |
| 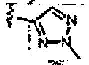 | CH₃ | Cl | F |
| 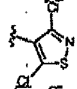 | CH₃ | Cl | F |
| 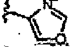 | CH₃ | Cl | F |
| 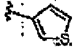 | CH₃ | Cl | F |
| 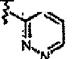 | CH₃ | Cl | F |
| 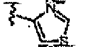 | CH₃ | Cl | F |
| 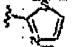 | CH₃ | Cl | F |
| 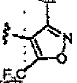 | CF₂H | Cl | F |
| 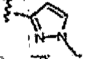 | CH₃ | Cl | F |
| 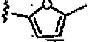 | CH₃ | Cl | F |
| 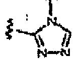 | CH₃ | Cl | F |
et sels pharmaceutiquement acceptables de celui-ci.

5. Composition pharmaceutique qui comprend une quantité thérapeutiquement efficace d'un composé selon l'une quelconque des revendications 1-4 ou d'un sel pharmaceutiquement acceptable de celui-ci et un véhicule pharmaceutiquement acceptable.

6. Utilisation d'un composé selon l'une quelconque des revendications 1-4 ou d'un sel pharmaceutiquement acceptable de celui-ci pour la fabrication d'un médicament pour le traitement d'états induits par le récepteur B1 de la bradykinine.
